# EUROPEAN PATENT SPECIFICATION

(11) **EP 2 526 425 B1**
(45) Date of publication and mention of the grant of the patent: **30.04.2014**
(21) Application number: 11700679.1
(22) Date of filing: 21.01.2011
(51) Int. Cl.: G01N 33/92, G01N 33/50

(54) **DIAGNOSTIC METHOD FOR ENDOMETRIAL RECEPTIVITY**
VERFAHREN ZUR DIAGNOSE DER ENDOMETRIALEN REZEPTIVITÄT
PROCÉDÉ DE DIAGNOSTIC DE LA RÉCEPTIVITÉ ENDOMÉTRIALE

(30) Priority: 21.01.2010 EP 10382011
(43) Date of publication of application: 28.11.2012
(73) Proprietor: Equipo Ivi Investigacion SL, 46015 Valencia (ES)
(72) Inventor: SIMÓN VALLÉS, Carlos, E-46015 Valencia (ES); PELLICER MARTÍNEZ, Antonio, E-46015 Valencia (ES); BERLANGA ATIENZA, Óscar, E-46015 Valencia (ES)
(74) Representative: Denjean, Eric
(86) International application number: PCT/EP2011/050867
(87) International publication number: WO 2011/089240

(56) References cited:
- HOOZEMANS DIEDERIK A ET AL: "Human embryo implantation: current knowledge and clinical implications in assisted reproductive technology", REPRODUCTIVE BIOMEDICINE ONLINE, REPRODUCTIVE HEALTHCARE LTD, GB, vol. 9, no. 6, 1 January 2004 (2004-01-01) , pages 692-715, XP008123049, ISSN: 1472-6483 [retrieved on 2004-10-15] cited in the application
- DATABASE EMBASE [Online] ELSEVIER SCIENCE PUBLISHERS, AMSTERDAM, NL; 2009, WASIELAK M ET AL: "Effect of the conceptus on uterine prostaglandin-F2[alpha] and prostaglandin-E2 release and synthesis during the periimplantation period in the pig", XP002622811, Database accession no. EMB-2009328756 & REPRODUCTION, FERTILITY AND DEVELOPMENT 2009 CSIRO AUS LNKD- DOI:10.1071/RD08236, vol. 21, no. 5, 2009, pages 709-717, ISSN: 1031-3613
- CHARTRAND R ET AL: "Effect of dietary fat sources on systemic and intrauterine synthesis of prostaglandins during early pregnancy in gilts.", JOURNAL OF ANIMAL SCIENCE, vol. 81, no. 3, March 2003 (2003-03), pages 726-734, XP002622812, ISSN: 0021-8812
- MAATHUIS J B: "CYCLIC CHANGES IN THE CONCENTRATION OF PROSTAGLANDIN F-2-ALPHA IN HUMAN UTERINE FLUSHINGS", BRITISH JOURNAL OF OBSTETRICS AND GYNAECOLOGY, vol. 85, no. 3, 1978, pages 207-210, XP002622813, ISSN: 0306-5456
- ACHACHE H ET AL: "Endometrial receptivity markers, the journey to successful embryo implantation", HUMAN REPRODUCTION UPDATE, OXFORD UNIVERSITY PRESS, OXFORD, GB, vol. 12, no. 6, 1 November 2006 (2006-11-01), pages 731-746, XP002587289, ISSN: 1355-4786, DOI: DOI:10.1093/HUMUPD/DML004 [retrieved on 2006-09-18]
- GEMZELL-DANIELSSON KRISTINA ET AL: "The effect of antiprogestin (RU 486) and prostaglandin biosynthesis inhibitor (naproxen) on uterine fluid prostaglandin F-2-alpha concentrations", HUMAN REPRODUCTION (OXFORD), vol. 9, no. 9, 1994, pages 1626-1630, XP008135258, ISSN: 0268-1161
- WOCLAWEK-POTOCKA ET AL: "Phytoestrogen metabolites are much more active than phytoestrogens themselves in increasing prostaglandin F2alpha synthesis via prostaglanin F2alpha synthase-like 2 stimulation in bovine endometrium", PROSTAGLANDINS AND OTHER LIPID MEDIATORS, ELSEVIER, US, vol. 78, no. 1-4, 1 December 2005 (2005-12-01), pages 202-217, XP005174176, ISSN: 1098-8823
- DATABASE BIOSIS [Online] BIOSCIENCES INFORMATION SERVICE, PHILADELPHIA, PA, US; 1994, MEYER HEINRICH H D: "Luteal versus placental progesterone: The situation in the cow, pig and bitch", XP002631531, Database accession no. PREV199497469780 & EXPERIMENTAL AND CLINICAL ENDOCRINOLOGY, vol. 102, no. 3, 1994, pages 190-192, ISSN: 0232-7384

## Description

### FIELD OF THE INVENTION

The invention relates to a non-invasive method for the detection of endometrial receptivity to embryo implantation. The method is especially applicable for determining status fertility of a mammalian female, preferably, a woman.

### BACKGROUND OF THE INVENTION

An estimated 12% of couples attempting to conceive suffer from infertility, and because of the increasing age of pregnancy this figure is on the rise. *In vitro* fertilisation (IVF) has been used ever more to assist infertile couples in becoming pregnant. Since the first successful IVF treatment in 1978 that led to the birth of Louise Brown, many technical and medical advances have contributed to improve efficiencies; nowadays, around 70% of women undergoing IVF treatment have successful outcomes. Still some 50% of patients do not become pregnant, or have early pregnancy loses, which calls for further efforts among the scientific community to continue research into the field.

IVF generally is a multistep process that involves recovering mature eggs from the female patient or donor; incubation of eggs in artificial culture media; collection of sperm from the patient or donor and subsequent preparation; fertilization of the egg by the sperm; monitoring and selection of good-quality embryos; and transfer of these to the uterine cavity. The embryo must implant onto the receptive endometrium so that pregnancy can progress. It is well established that this particular step is responsible for a significant percentage of the failures, and, therefore, it is clinically relevant to look at possible causes and solutions of the problem.

Implantation of the transferred embryo involves a synchronized crosstalk between a receptive endometrium and a functional blastocyst [Wang, H., and Dey, SK. 2006. Roadmap to embryo implantation: clues from mouse models. Mature Review Genetics. 7:185-199]. This phenomenon can only take place during the window of implantation, a self-limited period of endometrial receptivity spanning between days 19 and 23 of the menstrual cycle (women). In normal menstrual cycles this is achieved through the local effects of ovarian estrogens and progesterone, which induce a series of cellular and molecular events in the endometrium leading to appropriate endometrial receptivity. The latter is assessed using the Noyes criteria for endometrial dating, which was proposed over 50 years ago [Noyes, RW., Hertig, AJ., and Rock, J. 1950. Dating the endometrial biopsy. Fertil Steril. 1:3-25] but is still the preferred method used today to measure endometrial receptivity. Today, in the light of 15 years of experience with donor-egg IVF, it is accepted that histological evaluation adds little clinically significant information, and should be replaced by functional assessment of endometrial receptivity.

The era of molecular and cellular biology, and the development of new analytical techniques, has aided the quest for more consistent predictors of a receptive endometrium. Upon induction with steroid hormones, a large number of structural and molecular mediators have been identified to date that make potential biomarkers of endometrial receptivity, including the presence of pinopodes on the surface of endometrial cells, adhesion molecules such as integrins, cytokines, growth factors, lipids and others [Nikas, G., and Aghajanova. 2002. Endometrial pinopodes: some more understanding on human implantation? Reprod Biomed Online 4 Suppl. 3:18-23; Lessey, BA. 2003. Two pathways of progesterone action in the human endometrium: implications for implantation and contraception. Steroids. 68:809-815; Robb L, Dimitriadis E, Li R, Salamonsen LA., 2002. Leukemia inhibitory factor and interleukin-11: cytokines with key roles in implantation. J Reprod Immunol. 57: 129-141]. By illustrative, the production of two prostaglandins (PGs), namely prostaglandin E2 (PGE2) and prostaglandin F2 alpha (PGF2α), increases in human endometrium in the mid-luteal phase where the implantation window is situated [Hoozemans DA et al. Human embryo implantation: current knowledge and clinical implications in assisted reproductive technology. Reprod Biomed Online 2004, 9(6):692-715].

Furthermore, genetic and proteomic analysis have added new exciting tools for the assessment of endometrial receptivity, and endometrial biopsy samples can be used to identify molecules associated with uterine receptivity to obtain a better insight into human implantation. These approaches, however, have not yet rendered results applicable to the everyday-IVF clinical practice, since no single specific factor has been identified to be crucial for implantation in humans [Strowitzki, T., Germeyer, A., Popovici, R., et al. 2006. The human endometrium as a fertility-determining factor. Human Reproduction Update. 12:617-630]. Furthermore, a major setback in the application of biopsy-dependent techniques lies on their invasive nature, and also in the necessity of appropriately timed endometrial biopsies that disrupt the necessary endpoint of implantation [van der Gaast, MH., Beier-Hellwig, K., Fauser, BC., et al. 2003. Endometrial secretion aspiration prior to embryo transfer does not reduce implantation rates. Reprod Biomed Online. 7:105-109].

The analysis of endometrial secretions is a new, non-disruptive possibility for the investigation of endometrial receptivity. Importantly, the aspiration of endometrial fluid does not affect pregnancy rates [van der Gaast et al., 2003, cited *supra*]. This approach provides reliable read-outs of individual proteins correlating with day of cycle and has proven efficient in protein arrays using a luminex system [Boomsma, CM., Kavelaars, A., Eijkemans, MJC., et al. 2009. Cytokine profiling in endometrial secretions: a non invasive window on endometrial receptivity. Reprod Biomed Online. 18:85-94], thus opening the field for a non-invasive application.

Although some biochemical markers have been disclosed by assessing endometrial receptivity [Giudice, L.C. 1999. Hum Reprod 14 Suppl 2:3-16; Achache, H et al.; Human Reproduction Update, 2006; 12:731-46, Thomas, K et al.; Fertil. Steril. 2003; 80:502-507; WO03062832], there is a need in the art for specific and reliable molecular biomarkers for the detection of endometrial receptivity to embryo implantation in women, which can be used in clinic.

Metabolomics is a discipline dedicated to the systematic study of small molecules (i.e., metabolites) in cells, tissues, and biofluids. Metabolites are the end products of cellular regulatory processes, and their levels can be regarded as the amplified response of biological systems to genetic or environmental changes. Clinicians have relied for decades on a small part of the information contained in the metabolome, for example measuring glucose to monitor diabetes and measuring cholesterol for cardiovascular health. New sophisticated metabolomic analytical platforms and informatic tools have already been developed that afford extended and sensitive measurement of the metabolome.

Lipids are known to play an important role as structural components (e.g., cell membranes), energy storage components, and as signalling molecules. Lipids are broadly defined as hydrophobic or amphipathic small molecules that may originate entirely or in part by carbanion based condensation of thioesters, and/or by carbocation based condensation of isoprene units. "Lipidomics" can be considered as a sub-field of metabolomics which aims to elucidate the biological processes in the context of lipids by measuring and characterizing the extended lipid profiles at the molecular level (lipidomic profiles). Traditional clinical lipid measures quantify total amounts of triglycerides, cholesterol, or lipoproteins. However, serum lipid profile is more complex at the molecular level. Current lipidomics platforms enable quantitative characterization of hundreds of diverse lipid molecular species across multiple lipid classes such as sphingolipids, phospholipids, sterol esters, acylglycerols, sterols, bile acids, fatty acids, eicosanoids, prostaglandins, and steroids [Ore 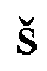 i 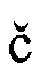 ,Seppaänen-Laakso, T and M. 2009. How to study lipidomes. *JMolec Endocr.* 42: 185-190].

### SUMMARY OF THE INVENTION

The invention relates to a method for detecting endometrial receptivity to embryo implantation in a mammalian female comprising the steps of:
a) determining the level of prostaglandin E2 (PGE2) in an endometrial fluid sample obtained from said mammalian female; and
b) identifying said mammalian female as receptive to embryo implantation when the level of PGE2 in said endometrial fluid sample is increased in relation to a reference sample.

In a particular embodiment, the level of prostaglandin F2 alpha (PGF2α) in the endometrial fluid sample obtained from said mammalian female is also determined.

Advantageously, said mammalian female is a woman.

According to an embodiment, the reference sample is endometrial fluid sample obtained from said female during non-fertile period of said female.

According to another embodiment the reference sample is endometrial fluid sample obtained from a population of mammalian females during non-fertile period of said females.

In a particular embodiment, the level of both PGE2 and PGF2α is measured.

In a particular embodiment, the levels of PGE2 and/or PGF2α are determined by liquid chromatography combined with tandem mass-spectrometry.

### BRIEF DESCRIPTION OF DRAWINGS

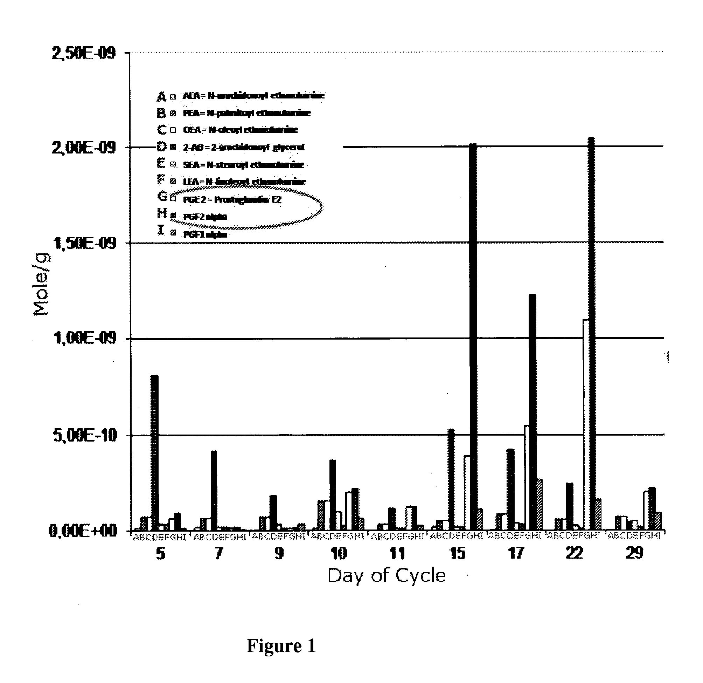

Figure 1 shows the levels of different lipids from endometrial fluid samples from women, during their natural menstrual cycle; the lipids were identified by liquid chromatography combined with tandem mass-spectrometry [A: N-arachidonoyl ethanolamine (AEA); B: N-palmitoyl ethanolamine (PEA); C: N-oleoyl ethanolamine (OEA); D: 2-arachidonoyl glycerol (2-AG); E: N-stearoyl ethanolamine (SEA); F: N-linoleoyl ethanolamine (LEA); G: prostaglandin E2 (PGE2); H: prostaglandin F2 alpha (PGF2α); I: prostaglandin F1 alpha (PGF1α)].

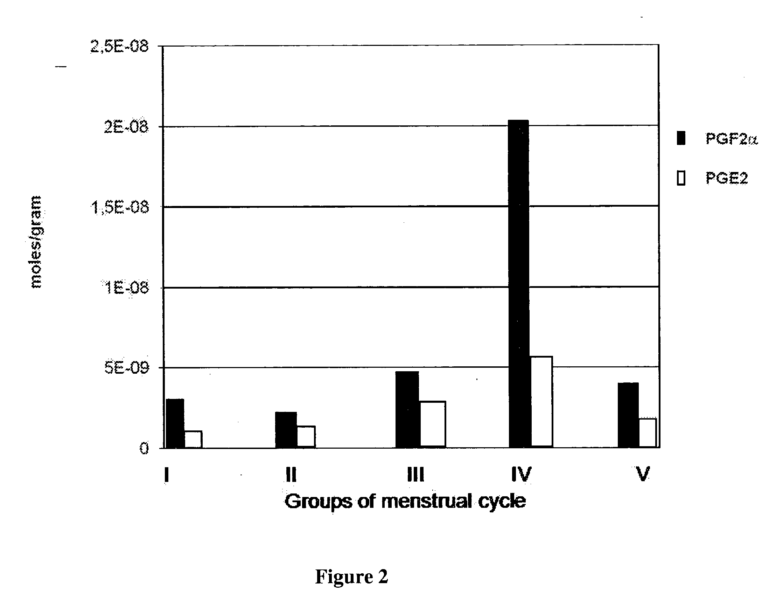

Figure 2 shows the levels of prostaglandin E2 (PGE2) and prostaglandin F2 alpha (PGF2α) in endometrial-fluid samples from women obtained throughout the menstrual cycle [Group I (days 0-8); Group II (days 9-14); Group III (days 15-18); Group IV (days 19-23) and Group V (days 24-30)].

### DETAILED DESCRIPTION OF THE INVENTION

An objective of the present invention is to provide a specific and reliable biomarker for the detection of endometrial receptivity for implanting an embryo in a woman. The biomarker can be used, for example, for detecting endometrial receptivity to embryo implantation in a mammalian female, or for selecting the implantation window of an embryo in a mammalian female, or for assessing the fertility status of a mammalian female, or for assessing if a mammalian female is under conditions suitable for receiving and implanting an embryo, or for monitoring endometrial maturation in a mammalian female, or in a method of *in vitro* fertilization in a mammalian female. In a particular embodiment, said mammalian female is a woman. In a further particular embodiment, said woman is a woman subjected to an *in vitro* fertilization (IVF) process.

The inventors have now surprisingly found that some lipid compounds can be used as biomarkers for endometrial receptivity for implanting an embryo. In particular, the inventors have analyzed the levels of some lipids in endometrial fluid samples between days 19 to 21 of the menstrual cycle of a woman, coincident with the implantation window. Thus, it is now possible to identify the implantation window in a mammalian female by determining the lipid profile in endometrial fluid samples along the menstrual cycle. The method is based on comparison of the established lipid profile from a mammalian female to reference lipidomic biomarkers. In a particular embodiment, said mammalian female is a woman.

In a particular embodiment, the inventors have observed that the concentration of prostaglandin PGE2 is significantly increased in endometrial fluid samples between days 19 to 21 of the menstrual cycle of a woman, coincident with the implantation window. Thus, it is now possible to identify the implantation window in a woman by determining the level of PGE2, possibly in combination with PGF2α, in endometrial fluid samples along the menstrual cycle.

PGE2, 7-[3-hydroxy-2-(3-hydroxyoct-1-enyl)-5-oxo-cyclopentyl]hept-5-enoic acid, is a naturally occurring prostaglandin, also known in medicine as "dinoprostone"; it has important effects in labour (softens cervix and causes uterine contraction) and also stimulates osteoblast to release factors which stimulate bone resoption by osteoclasts). Like other prostaglandins, dinoprostone can be used as an abortifacient since it is a direct vasodilator, relaxing smooth muscles, and it inhibits the release of noradrenaline from sympathetic nerve terminals.

PGF2α, (Z)-7-[(1R,2R,3R,5S)-3,5-dihydroxy-2-[(E,3S)-3-hydroxyoct-1-enyl) cyclopentyl]hept-5-enoic acid, is also a naturally occurring prostaglandin, also known in medicine as "dinoprost"; it is used in medicine to induce labour and as an abortifacient.

Thus, said prostaglandin PGE2, when determined in endometrial fluid samples, can be used as a biomarker of endometrial receptivity to embryo implantation in a mammalian female, preferably, a woman. Further, the use of said prostaglandin PGE2, in particular, when said prostaglandin, is determined in an endometrial fluid sample from a mammalian female, preferably, a woman, as a biomarker of endometrial receptivity to embryo implantation in said mammalian female, constitutes an aspect of this invention. A "biomarker", as used herein, refers to any lipid, for example, a prostaglandin, such as PEG2 and PGF2α, whose level (concentration) in endometrial fluid is altered in relation to a physiological condition of interest.

In a particular embodiment, the invention is based on the discovery that the level of a prostaglandin selected from the group consisting of prostaglandin PGE2, possibly in combination with PGF2α, in a sample of endometrial fluid from a mammalian female, e.g., a woman, can be used as a biomarker of endometrial receptivity to embryo implantation in a mammalian female, e.g., a woman.

As used herein, the term "endometrial fluid" refers to a secretion of the glandular epithelium which contains all the compounds secreted to the lumen of the uterus [Aplin JD et al. The Endometrium, 2nd. edn: INFORMA healtcare; 2008]. Briefly, the endometrium constitutes the surface tissue which lines the uterine wall (myometrium or middle layer of the uterine wall consisting of smooth muscle cells and supporting stromal and vascular tissue) of a mammalian female such as a woman or a human or non-human primate or, in other words, the inner membrane of the mammalian uterus. The endometrium is extremely sensitive to the hormones estrogen and progesterone and is composed of several functional layers. The tissue architecture of the endometrium comprises an external cell layer, which constitutes the epithelium, which is in direct contact with the lumen of the uterus, and an internal cell layer, denominated stroma, which constitutes around 80% of the endometrium thickness. Further, the endometrium contains blood vessels and specialized cells which confer its functional characteristics to the endometrium. The endometrial epithelium constitutes a continuous cell layer which is organized in two regions: luminal epithelium (epithelial cells that line the surface of the endometrium) and glandular epithelium (epithelial cells that form glands beneath the surface of the endometrium), said regions being physically different and molecularly recognizable [Brown SE et al. Endometrial glycodelin-A expression in the luteal phase of stimulated ovarian cycles. Fertil Steril 2000, 74(1):130-133]. The luminal epithelium represents a physical barrier against the pathogens attacks and develops structures, denominated pinopodes, in its apical surface, which are responsible of the absorption of uterine lumen material; however, the glandular epithelium is responsible for the secretions which form the endometrial fluid, which contains all compounds secreted to the lumen of the uterus [Aplin JD et al. (2008) cited *supra*].

The relevance of said secretions by endometrial glands is shown in a model of farm animals in which inhibition in the formation of said glands makes impossible the beginning of the pregnancy [Gray CA et al. Evidence that absence of endometrial gland secretions in uterine gland knockout ewes compromises conceptus survival and elongation. Reproduction 2002, 124(2):289-300]. Thus, it has been proposed that a deficient endometrial gland activity may cause failures in pregnancy although there are no direct evidences in humans [Burton GJ et al. Human early placental development: potential roles of the endometrial glands. Placenta 2007, 28 Suppl A:S64-69]. Thus, interestingly, the composition of the endometrial fluid depends on the endometrial gland activity instead of the endometrium as a whole, as it has been previously reported [van der Gaast et al. The feasibility of a less invasive method to assess endometrial maturation-comparison of simultaneously obtained uterine secretion and tissue biopsy. BJOG 2009, 116(2):304-312] - van der Gaast et al. show some proteins whose levels in endometrial tissue versus endometrial fluid is different and do not correspond each other.

Thus, the present section describes methods for detecting endometrial receptivity to embryo implantation in a mammalian female; methods for selecting the implantation window of an embryo in a mammalian female; methods for assessing if a mammalian female is under conditions suitable for receiving and implanting an embryo; methods for monitoring endometrial maturation; and methods for *in vitro* fertilization in a mammalian female. In a particular aspect, said mammalian female is a female human or non-human primate, preferably a woman. In a further particular aspect, said woman is a woman subjected to *an in vitro* fertilization (IVF) process. In another particular aspect, the methods comprise detecting the level of a prostaglandin selected from the group consisting of PGE2, possibly in combination with PGF2α, in an endometrial fluid sample from said mammalian female, where the level of said prostaglandin is correlated with endometrial receptivity to embryo implantation. In another aspect, the methods comprise detecting the level(s) of PGE2 and possibly PGF2α in an endometrial fluid sample from said mammalian female, in one or more endometrial fluid samples obtained from a plurality of stages of the menstrual cycle of a mammalian female. In an aspect, the methods are based upon the discovery that the levels of said prostaglandin PGE2 follow a temporal pattern in endometrial fluid during the menstrual cycle, and that the levels of said prostaglandin in said endometrial fluid are increased during the implantation window, i.e., a window of time during which the uterine endometrium is receptive to conception.

In one aspect, the invention relates to a method for detecting endometrial receptivity to embryo implantation in a mammalian female, hereinafter referred to as "method of the invention [1]", said method comprising the steps of:
a) determining the level of prostaglandin E2 (PGE2), in an endometrial fluid sample from said mammalian female; and
b) correlating the level of said prostaglandin in said endometrial fluid sample with endometrial receptivity to embryo implantation.

As used herein, the term "endometrial receptivity to embryo implantation", refers to the state of the endometrium during the window of endometrial receptivity. The term "window of endometrial receptivity" refers to the time period between days 19 to 21 of an idealized 28 day human menstrual cycle. Similar cycles are known for other mammals and it is within the ordinary skill in the art to adapt methods described herein to such cycles.

Further, the term "mammalian", as used herein includes any mammal, for example, humans and non-human primates, cattle, goats, sheeps, horses, pigs, dogs, etc. In a preferred particular embodiment said mammal is a woman. The term "woman" refers to a human female. In some embodiments, the mammalian female within the methods of the present invention is a woman and the stages of the menstrual cycle are selected from the group consisting of the early secretory phase and the mid-secretory phase.

According to the present invention, the levels of PGE2, possibly in combination with PGF2α, are determined in a sample of endometrial fluid from the mammalian female (e.g., woman) under study. The endometrial fluid sample can be taken from said mammalian female by conventional means. By illustrative, a sample of endometrial fluid from a woman can be obtained as mentioned in Example 1, i.e., by gently introducing transcervically an empty flexible catheter into the uterine cavity and gradually applying a suction with a syringe. Since one major challenge lies on the collection of samples of adequate purity, to prevent contamination by cervical mucus during catheter removal, the outer sheath of the embryo transfer catheter is advanced to an appropriate depth from the external cervical os, following the application of suction; then, cervical mucus is aspirated prior to the endometrial secretion aspiration. Further, although generally endometrial fluid does not contain cells which could affect the results, in an embodiment, once the endometrial fluid sample is extracted, the sample is subjected to centrifugation in order to remove any eventual cell component which could be present in the sample. A satisfactory number of endometrial fluid samples combined with good sample-collection practice will be sufficient to avoid false results.

In order to obtain a more reliable detection of the endometrial receptivity to embryo implantation in a mammalian female, it is preferable to determine the levels of PGE2 and/or PGF2α in the endometrial fluid samples along the menstrual cycle. Thus, the method of the invention [1] can be performed daily, or each 2, 3, 4, 5, 6, 7, 8, 9, 10, 12, 13, 14, 15, 16, 17, 18, 19, 20, 21, 22, 23, 24, 25, 26, 27 or 28 days, so that the expression profile of said prostaglandins PGE2 and/or PGF2α in endometrial fluid samples can be determined along the full menstrual cycle. Typically, in women, endometrial fluid samples can be obtained during the proliferative phase (days 1-14 of the menstrual cycle or before the LH peak), in the early secretory phase (days 15-19 of the menstrual cycle or days 1-5 after the LH peak), in the intermediate secretory phase (days 20-24 of the menstrual cycle or days 6-10 after the LH peak) and in the late secretory phase (days 25-28 of the menstrual cycle or days 11-14 after the LH peak). According to the instant invention, the levels of PGE2 and/or PGF2α in endometrial fluid during the endometrial receptivity to embryo implantation are the highest during the full menstrual cycle.

The determination of the endometrium receptivity to embryo implantation is particularly important in a lot of techniques, such as IVF, embryo transfer, etc., as well as for determining the optimum period for conception in couples who are trying to conceive in a natural way.

As used herein, the term "level" is intended broadly and can mean a quantitative amount (e.g., weight or moles), a semi-quantitative amount, a relative amount (e.g., weight % or mole % within class or a ratio), a concentration, and the like. In a particular embodiment, the levels of PGE2 and PGF2α are expressed in moles/gram of fluid [in that case, although samples were liquid, due to the small volume thereof, the samples were weighed and the concentrations of said compounds was expressed by reference to said weight - values were duly normalized].

The determination of the levels of PGE2 and/or PGF2α in the endometrial fluid sample can be determined by using any suitable method in view of the lipids to be detected and, optionally, quantified; illustrative, non-limitative, methods include thin layer chromatography (TLC), gas chromatography (GC), liquid chromatography (LC), mass spectrometry (MS), NMR spectrometry, etc., and combinations thereof (e.g., LC/MS or the like). In a particular embodiment, said lipids (PGE2 and PGF2α) were identified by LC combined with tandem MS [LC/MS/MS]. Tandem mass-spectrometers include triple quadrupole, ion trap, and quadrupole/time-of-flight instruments, among others. These instruments typically use quadrupole technology to isolate a compound based upon its molecular weight prior to collision activation (fragmentation) and mass analysis of the fragmented components. This means that the mixture must be purified only to the point that the sample applied to the mass spectrometer is free of other compounds with the same mass. This can often be accomplished with a liquid-liquid extraction from the tissue followed by solid phase extraction methods. Quadrupole technology provides approximately 1 amu resolution; improved isolation within the mass spectrometer is accomplished using TOF/TOF instruments, which permits much finer resolution.

Alternative methods for determining the level of PGE2 and PGF2α from biological samples have been described, see, e.g., Voyksner & Bush, who disclosed that thermospray HPLC/MS analysis of the metabolites of arachidonic acid, including prostaglandins PGE2 and PGF2α among others, proved to be sensitive and specific [Voyksner, R.D. & Bush, E.D. Determination of prostaglandins, and other metabolites of arachidonic acid by thermospray HPLC/MS using post column derivatization. Biomedical and Environmental Mass Spectrometry, Volume 14, Issue 5:213 - 220 (published online: 13 Apr 2005)]; Surrenti et al., who disclosed a rapid and practical method for the separation and quantitation of PGE2 in human gastric juice by high performance liquid chromatography (HPLC) [Surrenti C. et al., High Performance Liquid Chromatographic Method for Prostaglandin E2 Determination in Human Gastric Juice Without Derivatization. Journal of Liquid Chromatography & Related Technologies, Volume 7, Issue 12 , October 1984, pages 2409 - 2419]; and Bastani et al., who developed an analytical method based on LC with negative electrospray ionization (ESI) coupled to tandem mass spectrometric detection (LC/MS/MS) for the determination of PGF2α derivatives concentrations in different biological samples [Bastani, N.E., et al. Determination of 8-epi PGF2α concentrations as a biomarker of oxidative stress using triple-stage liquid chromatography/tandem mass spectrometry. Rapid Communications in Mass Spectrometry, Volume 23 Issue 18, pages 2885 - 2890 (published Online: 10 Aug 2009)]. Another suitable method of detecting, and optionally quantifying, lipids in a biological sample employs stable isotope tracers to label the lipids.

In a particular embodiment, PGE2 and PGF2α levels are measured using liquid chromatography (LC) combine with tandem mass-spectrometry (MS) [LC/MS/MS].

The levels of PGE2 and optionally PGF2α can be based on quantitative and/or semi-quantitative analysis. For example, semi-quantitative methods can be used to determine a level of a particular lipid (PGE2 and optionally PGF2α) metabolite(s) above a threshold value or to determine ratios of different lipid metabolites, without assigning an absolute or relative numerical value. Quantitative methods can be used to determine a relative or absolute amount of a particular lipid metabolite(s) in the biological sample.

In semi-quantitative methods, a threshold or cutoff value can be determined by any means known in the art, and is optionally a predetermined value. In particular embodiments, the threshold value is predetermined in the sense that it is fixed, for example, based on previous experience with the assay and/or a population of mammalian females (e.g., women). Alternatively, the term "predetermined" value can also indicate that the method of arriving at the threshold is predetermined or fixed even if the particular value varies among assays or may even be determined for every assay run.

In another aspect, the invention relates to a method for selecting the implantation window of an embryo in a mammalian female, hereinafter referred to as the "method of the invention [2]", which comprises determining the level of PGE2, or the level of both PGE2 and PGF2α, in a sample of endometrial fluid from said mammalian female, wherein said implantation window is selected when the level of at least one of said prostaglandins PGE2, and possibly of PGF2α in said sample is increased in relation to a reference sample.

As used herein, the expression "method for selecting", relates to a method for determining the probability of a mammalian female (e.g., a woman) of being in a period receptive for embryo implantation. The skilled person in the art will observe that said prediction cannot be correct for the 100% of the mammalian females (e.g., women) under study. However, said expression requires that the prediction method provides correct results for a statistically significant portion of mammalian females (e.g., women), what can be determined by using standard statistical techniques such as the confidence intervals determination, p value determination, t test of Student, or the Mann-Whitney test, as explained by Dowdy and Wearden, Statistics for Research, John Wiley & Sons, New York 1983. Suitable confidence intervals are, at least, 50%, at least 60%, at least 70%, at least 80%, preferably, at least 90%, or most preferable, at least 95%. Preferably, p values are 0.2, 0.1, or, most preferably 0.05.

As used herein, the term "implantation window" encompasses the window of time during which the uterine endometrium is receptive to the conceptus; in humans, this occurs in the secretory stage of the menstrual cycle. Implantation is defined as days 6-8 post the day of the luteinising hormone (LH) surge. The implantation window can be estimated on the basis of a regular menstrual pattern as approximately 7 days before the expected first day of the menstrual period, i.e., it corresponds, therefore, to days 19-21 of an ideal menstrual cycle of 28 days in humans. Similar cycles have been disclosed in other mammals, so that the method of the invention could be adapted to any mammalian female.

The levels of PGE2 and possibly of PGF2α are determined in a sample of endometrial fluid from the mammalian female under study. In a preferred particular embodiment, said mammalian female is a woman. The endometrial fluid sample can be taken from said mammalian female by conventional means as mentioned in connection with method of the invention [1]. Further, the determination of the level of PGE2 and possibly of PGF2α in the endometrial fluid sample can be determined by using any suitable method as discussed in connection with the method of invention [1]. In a particular embodiment, the levels of PGE2 and possibly of PGF2α are measured by using liquid chromatography (LC) combined with tandem mass-spectrometry (MS) [LC/MS/MS].

A cording to the method of the invention [2], the determination of the levels of PGE2 and optionally PGF2α needs to be correlated to the level of said prostaglandins in a reference sample. Effectively, the implantation window is selected when the level of at least one of said prostaglandins PGE2 and optionally PGF2α in the endometrial fluid sample analyzed is increased in relation to a reference sample. The level of a prostaglandin such as PGE2 or PGF2α in the endometrial fluid sample from the mammalian female under study is "increased in relation to" the level of said prostaglandins in a reference sample according to the instant invention, when the level of said prostaglandins in the endometrial fluid sample under study is at least, 1,1-fold, 1,5-fold, 5-fold, 10-fold, 20-fold, 30-fold, 40-fold, 50-fold, 60-fold, 70-fold, 80-fold, 90-fold, 100-fold or even more with respect to the reference sample.

As used herein, the term "reference sample", relates to an endometrial fluid sample obtained from a mammalian female, e.g., a woman, during the non-fertile period of said mammal. Due to the eventual variability which may exist among different mammalian females (e.g., women) as to the production of said prostaglandins during the non-fertile period, the reference sample can be typically obtained by combining the same amounts of samples of a population of mammalian females. In a particular embodiment, typical reference samples will be obtained from clinically well-documented women. In said samples, normal concentrations (i.e., reference concentrations) of the biomarker (PGE2 and optionally PGF2α) can be determined, for example, by establishing the average concentration on the reference population. In order to determine the biomarker reference concentration, some considerations should be born in mind, e.g., age, etc. By illustrative, the same amounts of a group of at least 2, at least 10, at least 100, preferably, more than 1,000 mammalian females, e.g., women, preferably sorted bearing in mind the above mentioned considerations (e.g., age, etc.) can be taken as the reference group.

In a particular embodiment, the reference sample is obtained from the endometrial fluid from a woman, or from a population of women, during the non-fertile period of said woman/women. Although the reference sample can be obtained at any day during the non-fertile period of said woman/women, in a particular embodiment, said reference sample is obtained before the 15^{th} day of the menstrual cycle, typically between days 5-11 of the menstrual cycle. The predetermined value can be calculated by using the endometrial fluid samples of the menstrual cycle stage as defined above.

Alternatively, in order to obtain a more reliable determination of the implantation window, it is preferable to determine the levels of PGE2 and optionally PGF2α along the menstrual cycle. Thus, the method of the invention [2] can be performed daily, or each 2, 3, 4, 5, 6, 7, 8, 9, 10, 12, 13, 14, 15, 16, 17, 18, 19, 20, 21, 22, 23, 24, 25, 26, 27 or 28 days, so that the expression profile of said prostaglandins PGE2 and/or PGF2α can be determined along the full menstrual cycle. Typically, in women, endometrial fluid samples can be obtained during the proliferative phase (days 1-14 of the menstrual cycle or before the LH peak), in the early secretory phase (days 15-19 of the menstrual cycle or days 1-5 after the LH peak), in the intermediate secretory phase (days 20-24 of the menstrual cycle or days 6-10 after the LH peak) and in the late secretory phase (days 25-28 of the menstrual cycle or days 11-14 after the LH peak). In this way, the optimal implantation window will correspond to the period of time in which the levels of PGE2 and optionally PGF2α are the highest during the full menstrual cycle.

In a particular embodiment, the reference sample is obtained from the same mammalian female (e.g., woman) under study during her non-fertile days in order to quantify the level(s) of said prostaglandins (PGE2 and optionally PGF2α); said information can be used as a reference value.

In another particular embodiment, the reference sample is obtained from a generalised mammalian female population (e.g., a population of women) during their non-fertile days and the level(s) of said prostaglandins (PGE2 and optionally PGF2α) are quantified and combined to establish the reference value.

According to the present invention, bioactive lipids in endometrial secretions are analysed to determine their relative levels and establish a correlation with implantation and pregnancy outcome.

Thus, in other aspect, the disclosure relates to a method for assessing the fertility status of a mammalian female wherein said method comprises:
- determining the level of PGE2 or the level of both PGE2 and PGF2α in a sample of endometrial fluid from said mammalian female, and
- correlating the level of said prostaglandin(s) PGE2 and optionally PGF2α with fertility status.

The levels of PGE2 and optionally PGF2α in a sample of endometrial fluid from the mammalian female under study, obtained as mentioned above, can be determined by using any suitable method as discussed in connection with the method of invention [1].

This method is aimed at assessing levels of said prostaglandin(s) PGE2 and optionally PGF2α in endometrial fluid samples during the implantation window in the endometrium of said mammalian female. The PGE2 and optionally PGF2α levels in endometrial fluid correlate with endometrial receptivity and likelihood of conception. Thus, according to this method, knowing the levels of PGE2 and optionally PGF2α in a sample of endometrial fluid from said mammalian female, it is possible to know the most fertile days of her menstrual cycle wherein the optimal conditions for getting pregnant are satisfied.

In a preferred particular aspect of this method, said mammalian female is a woman.

In other aspect, the disclosure relates to a method for assessing if a mammalian female is under conditions suitable for receiving and implanting an embryo, wherein said method comprises:
- determining the level of PGE2 or the level of both PGE2 and PGF2α in a sample of endometrial fluid from said mammalian female, and
- correlating the level of said prostaglandin(s) PGE2 and optionally PGF2α with the conditions for receiving and implanting an embryo.

The levels of PGE2 and optionally PGF2α in a sample of endometrial fluid from the mammalian female under study, obtained as mentioned above, can be determined by using any suitable method as discussed in connection with the method of invention [1]. This method is also aimed at assessing levels of said prostaglandin(s) PGE2 and optionally PGF2α during the implantation window in the endometrium of said mammalian female. The PGE2 and optionally PGF2α levels are correlated with the conditions of the receiving mammalian females for receiving and implanting an embryo and likelihood of conception. Again, according to this method, knowing the levels of PGE2 and optionally PGF2α in a sample of endometrial fluid from said mammalian female, it is possible to know the most suitable days for a mammalian female for receiving and implanting an embryo.

In a particular aspect, said mammalian female is a woman, preferably a woman subjected to an *in vitro* fertilization (IVF) process. In that case, the PGE2 and optionally PGF2α levels are correlated with the conditions of the receiving woman for receiving and implanting an embryo and likelihood of conception. The sample of endometrial fluid from said woman can be obtained at any time before implanting the embryo, e.g., from some minutes (e.g., 15 minutes) to some hours (e.g., 4-6 hours), or even some days (e.g., 1, 7, 14, or even more) before the embryo transfer. In a particular embodiment, this method is planned to be performed on the endometrial fluid obtained in a non-disruptive fashion 5-6 hours prior to embryo transfer.

As discussed above, the ability to identify specific lipid mediators in endometrial secretions that act as biomarkers of endometrial receptivity is of great importance to the IVF process. By eliminating the necessity to obtain endometrial biopsies, a stressful step for the woman is avoided. Furthermore, correct endometrial dating is inaccurately obtained, despite the development of non-invasive techniques such as ultrasound ecography or magnetic resonance, which only provide with morphological information of the uterus that has proven not reliable. The present invention provides a method for determining the biochemical characteristics of the uterus and an appropriate receptive endometrium a few hours prior to embryo transfer, thus increasing the chances for successful embryo implantation. Thus, by optimizing the endometrial factor, the implantation rates could be improved by at least 5%, typically, at least 10%.

In other aspect, the disclosure relates to a method for monitoring endometrial maturation in a mammalian female, said method comprising the steps of:
a) determining the level(s) of PGE2, and possibly of PGF2α in endometrial fluid samples from said mammalian female obtained from a plurality of stages of the menstrual cycle of said mammalian female; and
b) correlating the level(s) of said prostaglandin(s) PGE2 and optionally PGF2α with endometrial maturation.

Alternatively, according to this aspect, the method for monitoring endometrial maturation in a mammalian female comprises the steps of:
a) obtaining an endometrial fluid sample from said mammalian female;
b) determining the level(s) of PGE2, and possibly of PGF2α in said endometrial fluid sample from said mammalian female;
c) repeating steps a) and b) with endometrial fluid samples obtained from a plurality of stages of the menstrual cycle of said mammalian female; and
d) correlating the level(s) of said prostaglandin(s) PGE2 and optionally PGF2α with endometrial maturation.

The levels of PGE2 and optionally PGF2α in said samples of endometrial fluid from the mammalian female under study, obtained as mentioned above, can be determined by using any suitable method as discussed in connection with the method of invention [1].

According to this method, samples of endometrial fluid are obtained from a plurality of stages of the menstrual cycle of the mammalian female under study, for example, in the case of an idealized menstrual cycle of a woman, during the proliferative phase (days 1-14 of the menstrual cycle), in the early secretory phase (days 15-19 of the menstrual cycle), in the intermediate secretory phase (days 20-24 of the menstrual cycle) and in the late secretory phase (days 25-28 of the menstrual cycle), in order to be able to correlate the levels of said prostaglandins PGE2 and optionally PGF2α with the endometrial maturation, thus knowing the state of the endometrium along the menstrual cycle.

In a particular aspect, said mammalian female is a woman, e.g., a woman subjected to an IVF process.

Also disclosed is a method of *in vitro* fertilization in a mammalian female, said method comprising the steps of:
a) determining the level(s) of PGE2, and possibly of PGF2α in endometrial fluid samples from said mammalian female obtained from a plurality of stages of the menstrual cycle of said mammalian female;
b) correlating the level(s) of said prostaglandin(s) PGE2 and optionally PGF2α in one or more endometrial fluid samples of step b) with endometrial maturation; and
c) introducing an embryo into the uterus of said mammalian female when said endometrium is mature.

Alternatively, according to this aspect, the method of *in vitro* fertilization in a mammalian female comprises the steps of:
a) obtaining an endometrial fluid sample from said mammalian female;
b) determining the level(s) of PGE2 and possibly of PGF2α in said endometrial fluid sample from said mammalian female;
c) repeating steps a) and b) with endometrial fluid samples obtained from a plurality of stages of the menstrual cycle of said mammalian female;
d) correlating the level(s) of said prostaglandin(s) PGE2 and optionally PGF2α in one or more endometrial fluid samples of step c) with endometrial maturation; and
e) introducing an embryo into the uterus of said mammalian female when said endometrium is mature.

The levels of PGE2 and optionally PGF2α in said samples of endometrial fluid from the mammalian female under study, obtained as mentioned above, can be determined by using any suitable method as discussed in connection with the method of invention [1].

According to this method, samples of endometrial fluid are obtained from a plurality of stages of the menstrual cycle of the mammalian female under study, as discussed above, and once the endometrium is mature, an embryo is introduced into the uterus of said mammalian female.

In particular, said mammalian female is a woman, e.g., a woman subjected to an IVF process.

Thus, as it has been previously mentioned, the invention provides a number of non-invasive diagnostic methods, based on the identification of the lipid profile in the endometrial fluid during the implantation window. In a particular embodiment, said lipid profile comprises PGE2, possibly in combination with PGF2α.

The methods provided by the instant invention are expected to increase current implantation rates by, at least 5%, preferably, at least 10%. An improvement of implantation rates is of benefit to both the medical community and patients by providing with a better medical assistance with increased chances of successful outcomes. By increasing pregnancy rates, a significant percentage of patients who must now go through a second round of treatment will avoid such a need, thus avoiding the anxiety and economic problem that it may represent.

Further, the methods provided by the instant invention will provide the medical community with a powerful tool in the diagnosis of endometrial receptivity prior to embryo transfer in IVF treatments. Said methods will eliminate the necessity for invasive approaches, and will improve current implantation rates by offering real-time biochemical read-outs of endometrial receptivity only a few hours before the embryo transfer procedure. Patients consequently avoid upsetting biopsy-collection processes.

The following example illustrates the invention but is not intended to limit the scope of the invention.

### EXAMPLE 1

### The concentration of PGE2 and PGF2α is significantly increased during the window of implantation

### 1.1 Materials

HPLC-grade methanol and acetonitrile used for mass spectrometric studies were purchased from VWR international (Plainview, NY). HPLC grade water, mass spectrometry/HPLC grade acetic acid, formic acid, and ammonium acetate were purchased from Sigma-Aldrich (St. Louis, MO).

### 1.2 Methodology

**Design.** Inventors conducted a single-blind study in 39 healthy female donors after obtaining informed, signed consent. All samples were collected from women during their natural menstrual cycle; endometrial aspirates were assigned an identification number and preserved using standard methods prior analysis.

**Endometrial secretion aspiration procedure.** With the patient lying in lithotomy position, the cervix was cleansed after insertion of the speculum. An empty flexible catheter (Wallace, Smith Medical International) was gently introduced 6 cm transcervically into the uterine cavity and suction was gradually applied with a 10 ml syringe. To prevent contamination by cervical mucus during catheter removal, the outer sheath of the embryo transfer catheter was advanced to a depth of 4 cm from the external cervical os, following the application of suction. Cervical mucus was aspirated prior to endometrial secretion aspiration for within patient comparison, in order to verify whether the aspirates represented cervical mucus rather than endometrial secretions.

**Sample analysis.** Lipids from endometrial-fluid extracts were identified by liquid chromatography (LC) combined with tandem mass-spectrometry (MS). HPLC grade water was added to the samples to make a 30% organic solution. Lipids were partially purified on C18 solid phase extraction columns as previously described [Bradshaw, HB., Rimmerman, N., Krey, JF and Walker, JM. 2006. Sex and hormonal cycle differences in rat brain levels of pain-related cannabimimetic lipid mediators. Am J Physiol Regul Integr Comp Physiol. 291: R349-358]. In brief, each 500 mg column was conditioned with 5 ml methanol and 2.5 ml water followed by loading of the water/supernatant solution. Columns were then washed with 2 ml water and 1.5 ml 55% methanol. Compounds were eluted with 1.5 ml methanol. Eluants were vortexed at maximum speed prior to mass spectrometric analysis.

Tandem mass-spectrometers include triple quadrupole, ion trap, and quadrupole/time-of flight instruments, among others. These instruments typically use quadrupole technology to isolate a compound based upon its molecular weight prior to collision activation (fragmentation) and mass analysis of the fragmented components. This means that the mixture must be purified only to the point that the sample applied to the mass spectrometer is free of other compounds with the same mass. This can often be accomplished with a liquid-liquid extraction from the tissue followed by solid phase extraction methods. Quadrupole technology provides approximately 1 amu resolution; improved isolation within the mass spectrometer is accomplished using TOF/TOF instruments, which permit much finer resolution.

Specifically, rapid separation of analytes was obtained using 10 µl injections (Agilent 1100 series autosampler, Wilmington, DE) onto a Zorbax eclipse XDB 2.1 × 50 mm reversed phase column. Gradient elution (200 µl/min) was formed under pressure on a pair of Shimadzu (Columbia, Maryland) 10AdVP pumps. Mass spectrometric analysis was performed with an Applied Biosystems/MDS Sciex (Foster City, CA) API 3000 triple quadrupole mass spectrometer equipped with an electrospray ionization source. Levels of each compound were analyzed by multiple reactions monitoring (MRM) on the LC/MS/MS system.

**Mass spectrometric quantitation.** The quantitation of analytes was achieved using Analyst software (Applied Biosystems-MDS Sciex; Framingham MA), which quantifies the amount of analyte in the sample based upon a power fit of a linear regression of known concentrations of synthetic standards. Statistical differences were determined using ANOVA with post-hoc Fisher's LSD using a 95% confidence interval for the mean (SPSS software, Chicago, IL).

### 1.3 Results

A total of 39 endometrial fluid samples obtained throughout the menstrual cycle [Group I (days 0-8) (n=8); Group II (days 9-14) (n=8); group III (days 15-18) (n=8); Group IV (days 19-23) (n=8) and group V (days 24-30) (n=7)] were analysed for changes in lipid concentration in two single-blinded independent experiments.

In the first experiment (n=13), inventors found a significant increase in the concentration of two specific lipids (PGE2 and PGF2α) between days 19 to 21 of the menstrual cycle, coincident with the window of implantation. None of the remaining lipids identified in the samples [N-arachidonoyl ethanolamine, N-palmitoyl ethanolamine, N-oleoyl ethanolamine, 2-arachidonoyl glycerol, N-stearoyl ethanolamine, N-linoleoyl ethanolamine, PGF1α] underwent significant changes during the menstrual cycle (Figure 1).

A second experiment (n=26) confirmed the peak of the same lipids reported in the first experiment. The combined results from both experiments (n=39) demonstrate a 2-fold and 20-fold increase peak in the concentration of each lipid, respectively, during the clinical window of implantation (Figure 2).

These results suggest that PGE2 and/or PGF2α could be important biomarkers of human endometrial receptivity during the window of implantation

## Claims

1. A method for detecting endometrial receptivity to embryo implantation in a mammalian female comprising the steps of:
a) determining the level of prostaglandin E2 (PGE2) in an endometrial fluid sample obtained from said mammalian female; and
b) identifying said mammalian female as receptive to embryo implantation when the level of PGE2 in said endometrial fluid sample is increased in relation to a reference sample.

2. Method according to claim 1, wherein the level of prostaglandin F2 alpha (PGF2α) in the endometrial fluid sample obtained from said mammalian female is also determined.

3. Method according to any one of claims 1 or 2, wherein said mammalian female is a woman.

4. Method according to any one of claims 1 to 3, wherein the reference sample is endometrial fluid sample obtained from said female during non-fertile period of said female.

5. Method according to any one of claims 1 to 3, wherein the reference sample is endometrial fluid sample obtained from a population of mammalian females during non-fertile period of said females.

6. Method according to any one of the preceding claims, wherein the level of both PGE2 and PGF2α is measured.

7. Method according to any one of the preceding claims, wherein the levels of PGE2 and/or PGF2α are determined by liquid chromatography combined with tandem mass-spectrometry.

## Patentansprüche

1. Verfahren zum Nachweisen der Rezeptivität des Endometriums für eine Embryoimplantation bei einem weiblichen Säuger,
das die folgenden Schritte aufweist:
a) Bestimmen des Wertes von Prostaglandin E2 (PGE2) in einer Fluidprobe des Endometriums, die von dem weiblichen Säuger erhalten wurde; und
b) Identifizieren des weiblichen Säugers als für eine Embryoimplantation empfänglich, wenn der Wert für PGE2 in der Fluidprobe des Endometriums im Verhältnis zu einer Bezugsprobe erhöht ist.

2. Verfahren nach Anspruch 1,
wobei der Wert für Prostaglandin F2α (PGF2α) in der von dem weiblichen Säuger erhaltenen Fluidprobe des Endometriums ebenfalls bestimmt wird.

3. Verfahren nach einem der Ansprüche 1 oder 2,
wobei der weibliche Säuger eine Frau ist.

4. Verfahren nach einem der Ansprüche 1 bis 3,
wobei die Bezugsprobe eine Fluidprobe des Endometriums ist, die während eines unfruchtbaren Zeitraums von dem weiblichen Säuger erhalten wurde.

5. Verfahren nach einem der Ansprüche 1 bis 3,
wobei die Bezugsprobe eine Fluidprobe des Endometriums ist, die von einer Population von weiblichen Säugern während eines unfruchtbaren Zeitraums dieser weiblichen Säuger erhalten wurde.

6. Verfahren nach einem der vorstehenden Ansprüche,
wobei sowohl der Wert von PGE2 als auch von PGF2α gemessen wird.

7. Verfahren nach einem der vorstehenden Ansprüche,
wobei die Werte von PGE2 und/oder PGF2α durch Flüssigchromatografie in Kombination mit einer Tandem-Massenspektrometrie bestimmt werden.

## Revendications

1. Procédé de détection de la réceptivité endométriale pour l'implantation d'un embryon dans un mammifère femelle comprenant les étapes de :
a) détermination du taux de prostaglandine E2 (PGE2) dans un échantillon de fluide endométrial obtenu à partir dudit mammifère femelle ; et
b) identification dudit mammifère femelle comme récepteur pour l'implantation d'embryon quand le taux de PGE2 dans ledit échantillon de fluide endométrial est augmenté par rapport à un échantillon référence.

2. Procédé selon la revendication 1, dans lequel le taux de prostaglandine F2 alpha (PGF2α) dans l'échantillon de fluide endométrial obtenu dudit mammifère femelle est aussi déterminé.

3. Procédé selon l'une quelconque des revendications 1 ou 2, dans lequel ledit mammifère femelle est une femme.

4. Procédé selon l'une quelconque des revendications 1 à 3, dans lequel l'échantillon référence est un échantillon de fluide endométrial obtenu de ladite femelle durant une période d'infécondité de ladite femelle.

5. Procédé selon l'une quelconque des revendications 1 à 3, dans lequel l'échantillon référence est un échantillon de fluide endométrial obtenu d'une population de mammifères femelles durant une période d'infécondité desdites femelles.

6. Procédé selon l'une quelconque des revendications précédentes, dans lequel le taux de PGE2 et de PGF2α est mesuré.

7. Procédé selon l'une quelconque des revendications précédentes, dans lequel les taux de PGE2 et/ou PGF2α sont déterminés par chromatographie liquide combinée à une spectrométrie de masse en tandem.
